# EUROPÄISCHE PATENTANMELDUNG

(11) **EP 4 375 690 A1**
(43) Veröffentlichungstag der Anmeldung: **29.05.2024**
(21) Anmeldenummer: 22209318.9
(22) Anmeldetag: 24.11.2022
(51) Int. Cl.: G01R 33/54, A61B 5/00

(54) **STEUERUNG EINER OBJEKTTRANSPORTEINHEIT EINES UNTERSUCHUNGSSYSTEMS MITTELS BERÜHRBILDSCHIRM**

(71) Anmelder: Siemens Healthineers AG, 91301 Forchheim (DE)
(72) Erfinder: Helmecke, Sven, 90427 Nürnberg (DE); Kalnischkies, Bernd, 91056 Erlangen (DE)
(74) Vertreter: Siemens Healthineers Patent Attorneys

(57) **Zusammenfassung**

Ein Untersuchungssystem (1) mit einer Objekttransporteinheit (7) zum Transport eines zu untersuchenden Objekts in einen Untersuchungsbereich soll vereinfacht werden. Dazu wird ein Berührbildschirm (12) genutzt, der zur Steuerung einer Funktion und/oder zur Visualisierung eines Zustands der Untersuchungseinheit (z.B. MRT-System) ausgebildet ist. Der Berührbildschirm (12) weist ein virtuelles Bedienelement (13) auf, das manuell in eine Bewegung, Auslenkung oder Zielposition versetzbar ist. In Abhängigkeit davon wird die Objekttransporteinheit (7) zu dem oder weg von dem Untersuchungsbereich bewegt.

## Beschreibung

Die vorliegende Erfindung betrifft ein Untersuchungssystem mit einer Untersuchungseinheit, die einen Untersuchungsbereich aufweist, einer Objekttransporteinheit, mit der ein zu untersuchendes Objekt in den und/oder aus dem Untersuchungsbereich transportierbar ist, und einem Berührbildschirm, der zur Steuerung einer Funktion und/oder zur Visualisierung eines Zustands der Untersuchungseinheit ausgebildet ist. Darüber hinaus betrifft die vorliegende Erfindung ein entsprechendes Verfahren zum Betreiben eines Untersuchungssystems.

Vorrichtungen zur Magnetresonanztomographie, MRT, sind bildgebende Vorrichtungen, die ein starkes äußeres Magnetfeld nutzen, um die Kernspins eines zu untersuchenden Objekts auszurichten und sie durch Anlegen eines HF-Anregungspulses zur Präzession um die entsprechende Ausrichtung anzuregen. Die Präzession beziehungsweise der Übergang der Spins von diesem angeregten Zustand in einen Zustand mit geringerer Energie erzeugt als Antwort ein elektromagnetisches Wechselfeld, das über Empfangsantennen als MR-Signal detektiert werden kann.

Mit Hilfe von magnetischen Gradientenfeldern kann den Signalen eine Positionskodierung aufgeprägt werden, die es anschließend erlaubt, das erhaltene Signal einem Volumenelement des Untersuchungsobjekts zuzuordnen. Das empfangene Signal kann dann ausgewertet werden, um beispielsweise eine Bilddarstellung des Untersuchungsobjekts bereitzustellen.

Bei Magnetresonanztomographie-Systemen (MRT) ist es erforderlich, die darzustellende Anatomie in der Mitte des Magneten zu positionieren, um eine ausreichend gute Bildqualität sicherzustellen. Der Grund hierfür liegt darin, dass beispielsweise die Homogenität des Magnetfelds sowie die Linearität des Gradientensystems und auch die Linearität des ausgesendeten Hochfrequenzfelds in diesem Bereich günstig für die Bildgebung sind.

Die Definition des anatomischen Bereichs, welcher anschließend zur Erzeugung der Bilddaten in das sogenannte ISO-Zentrum des Magneten verschoben wird, erfolgt typischerweise manuell durch den Benutzer. Die Bedienung der Patientenliege, auf der der Patient platziert wird, ist unter anderem mit einem Drehrad möglich. Dieses Drehrad ist im Hinblick auf die Reinigbarkeit sehr unvorteilhaft. Außerdem trägt das Drehrad zu den Betriebskosten des Systems bei. Des Weiteren erfordert das Drehrad in der Regel auch einen Durchbruch durch die Magnetabdeckung des MRT-Systems.

Grundsätzlich können mit MRT-Systemen nicht nur menschliche und tierische Patienten untersucht werden. Vielmehr können MRT-Systeme auch zur Untersuchung nicht lebender Objekte, wie etwa Schaufelräder von Turbinen und vieles andere mehr, verwendet werden. Derartige Untersuchungsobjekte werden nachfolgend auch kurz Objekte genannt.

Die Aufgabe der vorliegenden Erfindung besteht darin, ein Untersuchungssystem zu finden, das im Hinblick auf Hygiene und Kosten ein vorteilhaftes Bedienelement zum Steuern einer Objekttransporteinheit besitzt.

Erfindungsgemäß wird diese Aufgabe gelöst durch ein Untersuchungssystem und ein Verfahren gemäß den unabhängigen Ansprüchen. Vorteilhafte Weiterbildungen der Erfindung ergeben sich aus den Unteransprüchen.

Entsprechend der vorliegenden Erfindung wird demnach ein Untersuchungssystem mit einer Untersuchungseinheit bereitgestellt, die einen Untersuchungsbereich aufweist. Das Untersuchungssystem ist generell dazu ausgebildet, ein Objekt zu untersuchen. Das Objekt kann beispielsweise ein menschlicher Patient oder auch ein technischer Gegenstand sein. Die Form der Untersuchung kann beliebig sein. Beispielsweise kann optische oder akustische Strahlung zur berührungslosen Untersuchung eingesetzt werden. Das Untersuchungssystem besitzt dazu eine spezielle Untersuchungseinheit, die wiederum einen eigenen Untersuchungsbereich aufweist. Das zu untersuchende Objekt muss in den Untersuchungsbereich der Untersuchungseinheit gebracht werden, um eine bestmögliche Untersuchung zu erzielen.

Das Untersuchungssystem besitzt hierfür eine Objekttransporteinheit, mit der das zu untersuchende Objekt in den und/oder aus den Untersuchungsbereich heraus transportierbar ist. Die Objekttransporteinheit kann beispielsweise ein Förderband mit einer Aufnahme des Objekts aufweisen. Falls das Objekt ein Patient ist, kann die Objekttransporteinheit eine Patientenliege aufweisen, mit der der Patient definiert in den Untersuchungsbereich transportiert werden kann. In der Regel ist es mit der Objekttransporteinheit beziehungsweise mit der Patientenliege auch möglich, den Patienten wieder aus dem Untersuchungsbereich herauszutransportieren.

Darüber hinaus besitzt das Untersuchungssystem einen Berührbildschirm, der auch als Touchscreen oder Touchpanel bezeichnet werden kann. Dieser Berührbildschirm ist zur Steuerung einer Funktion und/oder zur Visualisierung eines Zustands der Untersuchungseinheit ausgebildet. Der Berührbildschirm stellt gegebenenfalls eine Schnittstelle zur manuellen Steuerung der Untersuchungseinheit dar. Beispielsweise können mithilfe des Berührbildschirms gewisse Funktionen der Untersuchungseinheit aktiviert oder deaktiviert werden. Dazu sind beispielsweise geeignete virtuelle Bedienelemente auf dem Berührbildschirm zu berühren. Beispielsweise können mithilfe des Berührbildschirms Beleuchtungselemente der Untersuchungseinheit an- und abgeschaltet werden. Gegebenenfalls können auch Komfortfunktionen wie etwa die Belüftung bei einem MRT-System mithilfe des Berührbildschirms an- und abgeschaltet werden. Der Berührbildschirm kann zusätzlich oder alternativ für die Visualisierung eines oder mehrerer Zustände der Untersuchungseinheit genutzt werden. Beispielsweise können Fehlermeldungen der Untersuchungseinheit oder EKG-Daten des Patienten auf dem Berührbildschirm optisch dargestellt werden. Ebenso lassen sich beispielsweise die Positionen von Lokalspulen auf dem Berührbildschirm grafisch darstellen oder es können Workflows mittels des Berührbildschirms manuell gesteuert und ihre aktuellen Zustände dargestellt werden.

Erfindungsgemäß weist der Berührbildschirm ein virtuelles Bedienelement auf, das manuell in eine Bewegung, eine Auslenkung oder eine Zielposition versetzbar ist. Auf dem Berührbildschirm wird also das virtuelle Bedienelement dargestellt, und es kann beispielsweise mit dem Zeigefinger verschoben werden. Dieses Verschieben kann zu einer (jeweils vorgegebenen) spezifischen Bewegung, spezifischen Zielposition oder einer spezifischen Auslenkung führen. Der Bildschirm selbst oder die angeschlossene Sensorik kann einen oder mehrere Parameter dieser Bewegung, Zielposition oder Auslenkung registrieren. So kann beispielsweise eine Geschwindigkeit der Bewegung oder eine Amplitude der Auslenkung bzw. ein tatsächlicher Ort, d.h. Zielposition, des virtuellen Bedienelements registriert werden. Alternativ kann auch nur eine Zielposition registriert werden, an die das virtuelle Bedienelement hin verschoben wurde, ohne wie bei der Auslenkung Anfangs- und Zielposition kennen zu müssen.

Darüber hinaus ist bei dem erfindungsgemäßen Untersuchungssystem die Objekttransporteinheit in Abhängigkeit von der Bewegung, Auslenkung oder Zielposition des virtuellen Bedienelements zu dem oder weg von dem Untersuchungsbereich bewegbar. Dies bedeutet, dass zumindest ein Teil der Objekttransporteinheit entsprechend der Bedienung des virtuellen Bedienelements in den Untersuchungsbereich beziehungsweise aus ihm heraus bewegt wird. Im Falle einer Patientenliege als Objekttransporteinheit bewegt sich die eigentliche Liege in den Untersuchungsbereich, aber nicht der Fuß beziehungsweise der Antrieb der gesamten Patiententisches. Ebenso bewegt sich beispielsweise eine Aufnahme, in die beispielsweise das zu untersuchende technische Objekt aufgenommen ist, mittels eines Förderbands oder eines Linearmotors in den Untersuchungsbereich. Es bewegt sich in diesem Fall auch nicht die gesamte Transporteinheit einschließlich Förderband oder Linearmotor in den Untersuchungsbereich, allenfalls ein Abschnitt davon.

In vorteilhafter Weise ist es damit möglich, dass die Objekttransporteinheit mittels des Berührbildschirms manuell gesteuert wird. Neben dem Berührbildschirm ist daher kein zusätzliches Steuerelement wie etwa ein Drehrad notwendig, um die Objekttransporteinheit zumindest teilweise in den Untersuchungsbereich zu bewegen. Hierdurch kann auf ein zusätzliches Bedienelement, wie das Drehrad, verzichtet werden, wodurch die Herstellungskosten des Untersuchungssystems reduziert werden. Als besonderer Vorteil ist anzusehen, dass der Berührbildschirm mit nur für Statusanzeigen oder Steuerungen der Untersuchungseinheit dient, sondern auch zur Steuerung der Objekttransporteinheit (ggf. des Patiententisches).

In einem Ausführungsbeispiel ist vorgesehen, dass das virtuelle Bedienelement als ausschließlich linear aus einer Ausgangsposition heraus auslenkbares Grafikelement ausgebildet ist. Ausgehend von der Ausgangsposition kann das lineare Auslenken in eine Richtung und gegebenenfalls auch in die entgegengesetzte Richtung erfolgen. Beispielsweise kann so das Bedienelement als virtueller Schieberegler realisiert sein. Dabei lässt sich das virtuelle Bedienelement z.B. mittels des Fingers auf dem Berührbildschirm verschieben beziehungsweise auslenken. Gegebenenfalls ist eine Maximalauslenkung in eine oder in beide Richtungen vorgesehen, wobei diese vorzugsweise auf dem Berührbildschirm auch erkennbar gekennzeichnet ist. "Ausschließlich linear" kann aber auch bedeuten, dass mehrere lineare Bewegungsmöglichkeiten in unterschiedliche Richtungen aneinandergefügt sind. Beispielsweise kann ein lineares Teilstück vertikal und ein zweites anschließendes Teilstück horizontal ausgerichtet sein. Somit könnte eine Patientenliege eines MRT-Systems mithilfe des virtuellen Bedienelements zunächst nach oben in die Einfahrposition gefahren werden und anschließend horizontal in den MRT-Patiententunnel. Diese zwei Bewegungen mit um 90 Grad verschiedenen Bewegungsrichtungen können durch die Objekttransporteinheit beziehungsweise die Patientenliege auch automatisch durchgeführt werden, wenn das virtuelle Bedienelement nur in einer Richtung linear aus der Ausgangsposition herausbewegt wird. In diesem Fall bedeutet das Herausbewegen des virtuellen Bedienelements eben beispielsweise das automatische Anheben der Patientenliege bis zur Einfahrposition und das horizontale Weiterfahren in Richtung MRT-Patiententunnel. Das Auslenken des virtuellen Bedienelements von der Ausgangsposition in die entgegengesetzte Richtung kann dann beispielsweise das Herausfahren der Patientenliege aus dem MRT-Patiententunnel und gegebenenfalls das automatische Weiterfahren der Patientenliege nach unten in eine bequeme Ausstiegsposition bedeuten.

Bei einem alternativen Ausführungsbeispiel ist das virtuelle Bedienelement als ausschließlich rotatorisch aus einer Ausgangsposition heraus auslenkbares Grafikelement ausgebildet. Ein derartiges rotatorisch auslenkbares beziehungsweise drehbares Bedienelement bildet grafisch und bezüglich der Bediensystematik das bekannte Drehrad nach. Auf dem Berührbildschirm kann dazu ein virtuelles Drehrad dargestellt werden. Ein solches virtuelles Drehrad lässt sich beispielsweise mit dem Finger im Uhrzeigersinn und gegen den Uhrzeigersinn drehen. Auch hierbei kann eine Maximaldrehung in jede Richtung vorgesehen sein. Das rotatorische Auslenken des virtuellen Drehrads wird von einer Steuerung beziehungsweise der Objekttransporteinheit des Untersuchungssystems als Bewegungsbefehl in die entsprechende Richtung gewertet beziehungsweise umgesetzt. Auch hierbei lassen sich ähnliche Bewegungsmuster realisieren wie bei der linearen Verschiebung des Bedienelements. Insbesondere kann das Drehen des virtuellen Drehrads auch dazu führen, dass die Objekttransporteinheit beziehungsweise die Patientenliege zwei gekoppelte Bewegungen automatisch hintereinander ausführt, wie etwa eine Teilbewegung nach oben und eine Teilbewegung nach links beziehungsweise rechts.

Bei einem weiteren Ausführungsbeispiel ist vorgesehen, dass eine Geschwindigkeit der Objekttransporteinheit von einem Maß der Auslenkung des virtuellen Bedienelements abhängt. Dies bedeutet beispielsweise, dass die Geschwindigkeit der Objekttransporteinheit zunimmt, wenn die virtuelle Bedieneinheit ausgehend von der Ausgangsposition weiter ausgelenkt wird. Mit anderen Worten, je größer die Auslenkung ist, desto größer ist auch die Geschwindigkeit der Objekttransporteinheit. Dies gilt nicht nur für lineare, sondern auch für rotatorische Auslenkungen. Die Geschwindigkeit kann beispielsweise linear mit dem Maß der Auslenkung zusammenhängen. Gegebenenfalls kann aber auch ein anderer funktioneller Zusammenhang zwischen Auslenkung und Geschwindigkeit bestehen, wie etwa ein quadratischer oder exponentieller. Vorzugsweise sind sowohl eine Vor- als auch eine Zurückbewegung der Objekttransporteinheit mit unterschiedlicher Geschwindigkeit dadurch realisierbar, dass das virtuelle Bedienelement entsprechend weit in die eine Richtung beziehungsweise in die andere entgegengesetzte Richtung ausgelenkt wird.

Darüber hinaus kann ein Ausführungsbeispiel vorgesehen sein, bei dem das virtuelle Bedienelement aus einer Auslenkung automatisch in die Ausgangsposition zurückgeht, wenn das virtuelle Bedienelement nicht mehr manuell berührt wird. Dies bedeutet, dass das virtuelle Bedienelement quasi durch eine Rückstellfeder in die Ausgangsposition zurückbewegt wird, wenn es nicht mehr manuell "gehalten" wird. Dies hat den Vorteil, dass das virtuelle Bedienelement beispielsweise nur dann fährt, wenn das virtuelle Bedienelement auch bewusst ausgelenkt wird. Das Rückstellen des virtuellen Bedienelements kann sprunghaft oder mit einem sanften Übergang erfolgen, bei dem die Geschwindigkeit der Objekttransporteinheit allmählich auf null abnimmt. Auf diese Weise kann beispielsweise automatisch erreicht werden, dass eine Patientenliege eines MRT-Systems stoppt, wenn die Bedienperson den Berührbildschirm beziehungsweise das virtuelle Bedienelement nicht mehr berührt.

Bei einem besonders bevorzugten Ausführungsbeispiel ist der Berührbildschirm direkt an der Untersuchungseinheit angebracht. Beispielsweise kann der Berührbildschirm bei einem MRT-System im Einfahrbereich der Röhre, d.h. am Gehäuse des Magnetsystems, angebracht sein. Dabei kann der Berührbildschirm trennbar oder untrennbar mit der Untersuchungseinheit verbunden sein. Die untrennbare Verbindung hat den Vorteil, dass der Berührbildschirm an einer festen Stelle stets erreichbar ist. Die trennbare Verbindung mit der Untersuchungseinheit hingegen kann den Vorteil bieten, dass die Objekttransporteinheit durch den Berührbildschirm ferngesteuert werden kann. Dazu ist der Berührbildschirm vorzugsweise durch eine drahtlose Datenverbindung mit der Untersuchungseinheit beziehungsweise der Objekttransporteinheit verbunden. Beispielsweise eignen sich hierzu entsprechende Bluetooth- beziehungsweise WLAN-Verbindungen.

Bei dem Untersuchungssystem kann es sich vorteilhafterweise um ein bildgebendes Untersuchungssystem handeln. Das bildgebende Untersuchungssystem besitzt dabei ein Untersuchungssystem nach obiger Art, wobei die Untersuchungseinheit dazu ausgebildet ist, von dem zu untersuchenden Objekt in dem Untersuchungsbereich ein Bild zu gewinnen. Bildgebende Untersuchungssysteme sind beispielsweise MRT-Systeme, CT-Systeme (Computertomographie), Angiographie-Systeme oder dergleichen. Mit dem virtuellen Bedienelement auf dem Berührbildschirm ist es dann möglich, das zu untersuchende Objekt in den Untersuchungsbereich zu fahren, wobei kein zusätzliches Drehrad neben dem Berührbildschirm erforderlich ist.

Die oben geschilderte Aufgabe wird erfindungsgemäß auch gelöst durch ein Verfahren zum Betreiben eines Untersuchungssystems obiger Art, wobei das virtuelle Bedienelement des Berührbildschirms manuell in eine Bewegung, eine Zielposition oder eine Auslenkung versetzt wird, und die Objekttransporteinheit in Abhängigkeit von der Bewegung, Zielposition oder Auslenkung des virtuellen Bedienelements zu dem oder weg von dem Untersuchungsbereich bewegt wird. Das manuelle Bedienen des virtuellen Bedienelements auf dem Berührbildschirm kann beispielsweise mittels eines Fingers einer Bedienperson erfolgen. Alternativ kann das manuelle Bedienen beispielsweise auch mithilfe eines Stifts oder eines anderen Hilfselements vollzogen werden.

Die oben im Zusammenhang mit dem Untersuchungssystem genannten Vorteile und Ausführungsvarianten gelten sinngemäß auch für das erfindungsgemäße Verfahren. Dementsprechend können die funktionellen Merkmale des Untersuchungssystems als entsprechende Verfahrensmerkmale gesehen werden.

Für Anwendungsfälle oder Anwendungssituationen, die sich bei dem Verfahren ergeben können und die hier nicht explizit beschrieben sind, kann vorgesehen sein, dass gemäß dem Verfahren eine Fehlermeldung und/oder eine Aufforderung zur Eingabe einer Nutzerrückmeldung ausgegeben und/oder eine Standardeinstellung und/oder ein vorbestimmter Initialzustand eingestellt wird.

Die vorliegende Erfindung wird nun anhand der beigefügten Zeichnungen näher erläutert, in denen zeigen:
- FIG 1: eine schematische Ansicht eines Untersuchungssystems gemäß der vorliegenden Erfindung;
- FIG 2: einen Berührbildschirm mit virtuellem Bedienelement;
- FIG 3: eine manuelle Auslenkung des virtuellen Bedienelements und
- FIG 4: ein Ablaufdiagramm eines beispielhaften Verfahrens.

Die nachfolgend im Zusammenhang mit den Zeichnungen näher erläuterten Ausführungsbeispiele stellen bevorzugte Ausführungsformen der vorliegenden Erfindung dar.

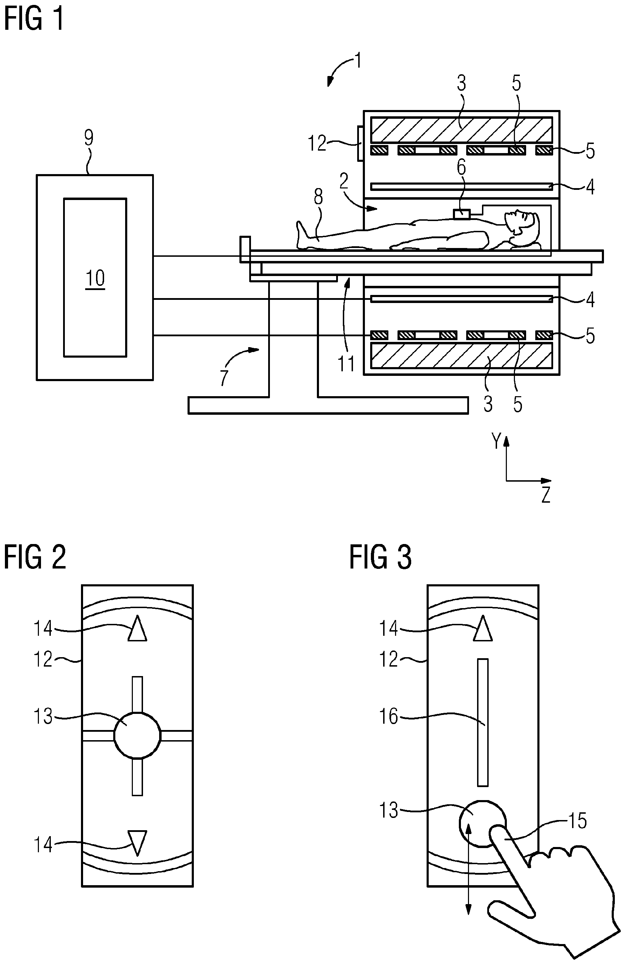

FIG 1 zeigt eine schematische Darstellung einer beispielhaften Ausführungsform eines erfindungsgemäßen (MRT-) Systems 1.

Das MRT-System 1 umfasst eine Magneteinheit mit einem Feldmagneten 3, der ein statisches Magnetfeld zur Ausrichtung von Kernspins eines Objekts 8, zum Beispiel eines Patienten, in einem Bildgebungsbereich erzeugt. Der Bildgebungsbereich bzw. Untersuchungsbereich ist durch ein äußerst homogenes statisches Magnetfeld gekennzeichnet, wobei sich die Homogenität insbesondere auf die Magnetfeldstärke beziehungsweise deren Amplitude bezieht. Der Bildgebungsbereich befindet sich in einem Patiententunnel 2, der sich in einer Längsrichtung Z durch die Magneteinheit erstreckt. Der Feldmagnet 3 kann beispielsweise ein supraleitender Magnet sein, der Magnetfelder mit einer magnetischen Flussdichte von bis zu 3 T oder mehr erzeugen kann. Für geringere Feldstärken können jedoch auch Permanentmagnete oder Elektromagnete mit normalleitenden Spulen verwendet werden. Eine hier als Patiententisch 7 ausgebildete Objekttransporteinheit besitzt eine Patientenliege 11, die in den Patiententunnel 2 bewegt werden kann, um den zu untersuchenden Teil des Patienten (Objekt) in den Untersuchungsbereich innerhalb des Patiententunnels 2 zu transportieren.

Das Untersuchungssystem beziehungsweise MRT-System 1 besitzt einen Berührbildschirm 12 (Touchscreen beziehungsweise Touchpanel) beispielsweise an der Abdeckung der Magneteinheit etwa am Eingang in den Patiententunnel. Dieser Berührbildschirm 12 ist dazu ausgebildet, Zustandsdaten des Untersuchungssystems darzustellen. Dementsprechend kann beispielsweise visualisiert werden, ob eine Beleuchtung oder eine Belüftung in dem Patiententunnel 2 eingeschaltet ist. Außerdem können an dem Berührbildschirm 12 für die Untersuchung relevante Daten dargestellt werden. Solche Daten kann der Berührbildschirm 12 beispielsweise von einem Steuer-und Rechensystem 9 empfangen. Außerdem kann der Berührbildschirm 12 gegebenenfalls auch Workflow-Daten darstellen, die er ebenfalls von dem Steuer- und Rechensystem 9 empfängt. Vorzugsweise besteht eine bidirektionale Datenverbindung zu dem Steuer- und Rechensystem 9, sodass mithilfe des Berührbildschirms 12 Steuerbefehle für das Untersuchungssystem manuell eingegeben werden können.

Des Weiteren besitzt der Berührbildschirm 12 eine Datenverbindung zu der Objekttransporteinheit, die hier als Patiententisch 7 realisiert ist. Diese Datenverbindung kann drahtgebunden oder drahtlos sein. Über diese Verbindung ist es möglich, Bewegungen einer Objektaufnahme (hier Patientenliege 11) des Patiententisches 7 mit dem Berührbildschirm zu steuern. Dadurch ergibt sich eine doppelte Funktionalität des Berührbildschirms, nämlich Funktionen in Bezug auf die Untersuchungseinheit aber auch die Objekttransporteinheit.

Weiterhin umfasst die Magneteinheit eine Gradientenspulenanordnung 5 mit mehreren Gradientenspulen, die dazu dienen, dem statischen Magnetfeld Gradientenfelder, also ortsabhängige Magnetfelder, in den drei Raumrichtungen zur räumlichen Differenzierung der abgetasteten Bildbereiche im Bildgebungsbereich zu überlagern. Die Gradientenspulen der Gradientenspulenanordnung 5 können zum Beispiel als Spulen aus normalleitenden Drähten ausgebildet sein, die zum Beispiel zueinander orthogonale Felder oder Feldgradienten im Bildgebungsbereich erzeugen können.

Die Magneteinheit umfasst eine Sendespulenanordnung, die beispielsweise eine Körperspule 4 als Sendeantenne umfassen kann, die dazu ausgebildet ist, ein Hochfrequenzsignal in den Bildgebungsbereich abzustrahlen. Die Körperspule 4 kann daher als HF-Sendespulenanordnung des MRT-Systems 1 verstanden werden oder als Teil der HF-Sendespulenanordnung. Die Körperspule 4 kann in einigen Ausführungsformen auch dazu verwendet werden, resonante MR-Signale zu empfangen, die von dem Objekt 8 ausgesendet werden. In diesem Fall kann die Körperspule 4 auch als Teil einer Signalerfassungsvorrichtung des MRT-Systems 1 betrachtet werden. Optional umfasst die Signalerfassungsvorrichtung eine lokale Spule 6, die in unmittelbarer Nähe des Objekts 8, zum Beispiel an dem Objekt 8 oder in dem Patiententisch 7, angeordnet sein kann. Die lokale Spule 6 kann alternativ oder zusätzlich zur Körperspule 4 als Empfangsspule beziehungsweise Empfangsantenne dienen.

Das MRT-System 1 umfasst auch ein Steuer- und Rechensystem 9. Das Steuer- und Rechensystem 9 kann eine Sende-Empfangssteuereinheit 10 umfassen, die mit der Körperspule 4, der Gradientenspulenanordnung 5 und/oder der lokalen Spule 6 verbunden ist. In Abhängigkeit von den erfassten MR-Signalen kann die Sende-Empfangssteuereinheit 10, die einen Analog-Digital-Wandler, ADC (englisch: "analog-to-digital converter") umfassen kann, entsprechende MR-Daten, insbesondere im k-Raum, erzeugen. Die Sende-Empfangssteuereinheit 10 ist gegebenenfalls auch mit der Körperspule 4 verbunden und steuert diese zur Erzeugung von HF-Impulsen, wie Anregungsimpulsen und/oder Refokussierungsimpulsen, an. Weiterhin kann die Sende-Empfangssteuereinheit 10 des Steuer- und Rechensystems 9 auch mit der Gradientenspulenanordnung 5 verbunden sein und diese steuern, um Schichtselektionsgradienten, Gradienten für die Frequenz- und/oder Phasencodierung und/oder Auslesegradienten zu schalten.

Das Steuer- und Rechensystem 9 kann die MR-Daten auswerten und zum Beispiel eine Bildrekonstruktion oder Teile davon oder anderweitige Rechenaufgaben übernehmen, die im Zuge der Bildgebung notwendig sind. Eine digitale Signalverarbeitungseinrichtung kann Teil der Sende-Empfangssteuereinheit 10 und damit auch Teil des Untersuchungssystems (hier MRT-System 1) sein.

Es wird darauf hingewiesen, dass die beschriebene Struktur des Steuer- und Rechensystems 9 nur ein nicht begrenzendes Beispiel darstellt. Die verschiedenen erforderlichen Aufgaben und Funktionen können auch anders und/oder auf verschiedene Steuereinheiten und/oder andere Recheneinheiten verteilt sein.

Das MRT-System 1 ist insbesondere in der Lage, ein erfindungsgemäßes Verfahren zum Betreiben eines Untersuchungssystems durchzuführen.

Wie oben bereits angedeutet wurde, ist es wichtig, den zu untersuchenden Bereich eines Objekts beziehungsweise Patienten in den örtlich begrenzten Untersuchungsbereich des Untersuchungssystems zu transportieren. In diesem Zusammenhang erfolgt die Definition des anatomischen Bereichs, welcher anschließend zur Erzeugung der Bilddaten in das sogenannte ISO-Zentrum (Untersuchungsbereich) des Magneten (bei einem MRT-System) verschoben wird, typischerweise manuell durch den Benutzer. Dies bedeutet beispielsweise, dass der Patient mehr oder weniger weit in den Patiententunnel 2 geschoben wird.

Die Bedienung des Patiententisches 7 zur Bewegung von dessen Patientenliege 11 erfolgt bei einem hier nicht beanspruchten Beispiel mit einem Drehrad. Ein solches Drehrad ist jedoch aus Gründen der Reinigbarkeit sehr unvorteilhaft. Außerdem trägt das Drehrad zu erhöhten Herstellungs- und Betriebskosten des Systems bei. Durch Weglassen des Drehrads können sowohl Hygiene- als auch Kostenaspekte optimiert werden.

Zur Lösung dieses Problems kann der oben genannte Berührbildschirm als Bedieninterface zur Verfügung gestellt werden. Damit kann beispielsweise bei einem MRT-System die Patientenliege mittels des Berührbildschirms verschoben werden. Dies bedeutet, dass der Berührbildschirm neben verschiedenen anderen Nicht-Liegen-bezogenen Funktionen die Möglichkeit bietet, die Patientenliege 11 z.B. auf bestimmte vordefinierte Zielpositionen zu schicken. Diese Art der Liegenbewegung nennt sich auch "Target Table Move". Im Gegensatz dazu bietet das oben genannte Drehrad auch die Möglichkeit, die Patientenliege 11 zu verfahren, solange das Drehrad in die eine oder andere Richtung ausgelenkt wird, bis die gewünschte Endposition erreicht ist. Diese Art der Liegenbewegung nennt sich "Continuous Table Move". Erfindungsgemäß wird sie auch mit dem Berührbildschirm realisiert.

Der Berührbildschirm bietet aufgrund der freien Programmierbarkeit die Möglichkeit, weitere Touch-Bedienflächen oder Schalter beziehungsweise virtuelle Bedienelemente zu integrieren. Beispielsweise kann auf dem Berührbildschirm 12 ein virtuelles Bedienelement 13 ähnlich einem Schieberegler implementiert werden, wie es in FIG 2 dargestellt ist. Pfeile 14 auf dem Berührbildschirm 12 können andeuten, in welche Richtung das virtuelle Bedienelement 13 bewegt werden kann. Im vorliegenden Beispiel soll das virtuelle Bedienelement 13 vorzugsweise nach oben oder unten geschoben werden können. Dies schließt jedoch nicht aus, dass das virtuelle Bedienelement 13 auch nach links und rechts verschoben werden kann, wenn beispielsweise die Patientenliege auch in X-Richtung, das heißt horizontal quer zur Längsrichtung des Patiententunnels 2, bewegbar ist.

FIG 3 deutet an, wie das virtuelle Bedienelement 13 manuell verschoben werden kann. Hierzu berührt beispielsweise der Finger 15 das virtuelle Bedienelement 13 auf dem Berührbildschirm 12 und führt es z.B., wie in FIG 3 dargestellt, unter fortlaufender Berührung des Berührbildschirms 12 nach unten. Dadurch könnte der Steuerbefehl ausgelöst werden, dass die Patientenliege 11 aus dem Patiententunnel 2 herausfährt. Umgekehrt könnte mit einer Bewegung des virtuellen Bedienelements 13 nach oben der Befehl erzeugt werden, dass die Patientenliege 11 in den Patiententunnel 2 gefahren wird. Das virtuelle Bedienelement 13 lässt sich also aus einer Ausgangsposition 16 heraus verschieben, wodurch der sogenannte Continuous Table Move ausgelöst wird. Optional kann diese manuell gesteuerte Bewegung dann abgebrochen werden, wenn das virtuelle Bedienelement 13 nicht mehr berührt wird. In diesem Fall bekommt der Patiententisch 7 auch keine entsprechenden Fahrbefehle mehr.

Bei einer Weiterbildung kann vorgesehen sein, dass sich über die Auslenkung des virtuellen Bedienelements 13 die Verfahrgeschwindigkeit der Objekttransporteinheit beziehungsweise der Patientenliege 11 ändern lässt. Wird das virtuelle Bedienelement 13 beispielsweise weit nach unten gezogen, so wird die Patientenliege entsprechend mit hoher Geschwindigkeit z.B. aus dem Patiententunnel herausgefahren. Demgegenüber kann die Geschwindigkeit beim Herausfahren entsprechend geringer sein, wenn das virtuelle Bedienelement 13 nur geringfügig aus der Ausgangsposition 16 heraus nach unten gezogen wird. Ähnliches gilt für entsprechende Auslenkungen beziehungsweise Auslenkungsamplituden nach oben.

Bei bestimmten MRT-Systemen ist vorgesehen, dass die Patientenliege beim Einfahren in den Patiententunnel 2 zunächst nach oben (Y-Richtung) bis zu einer vordefinierten Einfahrhöhe gefahren wird und anschließend horizontal (Z-Richtung) in den Patiententunnel 2 hinein. Dies kann in dem Patiententisch 7 als zusammenhängende Gesamtbewegung programmiert sein. Dementsprechend kann eine derartige kombinierte Y-Z-Bewegung auch durch eine einzige Auslenkung des virtuellen Bedienelements 13 aus der Ausgangsposition 16 heraus z.B. nach oben ausgelöst werden. Dies bedeutet, dass der Bediener unverändert das virtuelle Bedienelement 13 oberhalb der Ausgangsposition 16 hält und die Patientenliege 11 fährt zunächst hoch in Y-Richtung und bei Erreichen der Einfahrhöhe fährt sie automatisch in Z-Richtung weiter in den Patiententunnel 2. Alternativ kann eine derartige kombinierte Bewegung aber auch beispielsweise durch ein kombiniertes Führen des virtuellen Bedienelements 13 z.B. nach oben und anschließend nach rechts erzielt werden. Dabei kann das virtuelle Bedienelement 13 beim Beenden der ersten Bewegung in Y-Richtung zur Ausgangsposition 16 zurückspringen, was jedoch nicht zwingend erforderlich ist.

Durch die Möglichkeit, die Continuous Table Move-Liegenbewegungen mit dem Berührbildschirm 12 anzusteuern, kann auf ein Drehrad verzichtet werden. Hierdurch ergeben sich zahlreiche Vorteile. Zum einen kann eine Kostenersparnis erzielt werden, da der Drehknopf als solcher einschließlich der notwendigen Infrastruktur wie Kabel, BUS-Anbindung und Spannungsversorgung, nicht benötigt wird. Unter Umständen kann so an einem System, das z.B. bis zu drei Drehknöpfe für verschiedene Bedienorte hatte, ein entsprechend hoher Betrag eingespart werden.

Ein weiterer wesentlicher Vorteil besteht darin, dass bei einem MRT-System die Magnetabdeckung wesentlich vereinfacht werden kann, denn es ist keine Durchführung für den Drehknopf notwendig. Durch das vereinfachte Design der Magnetabdeckung können ebenfalls Kosten eingespart werden.

Ein weiterer Vorteil besteht darin, dass aufgrund des Verzichts auf einen oder mehrere Drehknöpfe Fertigungszeit eingespart werden kann. Zudem ergeben sich auch Vorteile hinsichtlich der Hygiene, denn Drehknöpfe sind deutlich schwerer zu reinigen als ebene Flächen.

Nebenbei ermöglicht die zu implementierende Ansteuerung über den Berührbildschirm auch die Möglichkeit, die Continuous Table Move-Liegenbewegung auch von einem Remote-System anzustoßen. Von dem Remote-System wird dann praktisch die Auslenkung des virtuellen Bedienelements simuliert, um das entsprechende Steuerungskommando zu erzeugen.

Es besteht auch die Möglichkeit, dass der Berührbildschirm ein erweitertes Display eines Bediencomputers darstellt, der in einem Vorbereitungsraum des Untersuchungssystems angeordnet ist. In diesem Fall könnte die Steuerung der Objekttransporteinheit beziehungsweise des Patiententisches direkt vom Bediencomputer aus oder über den Berührbildschirm erfolgen.

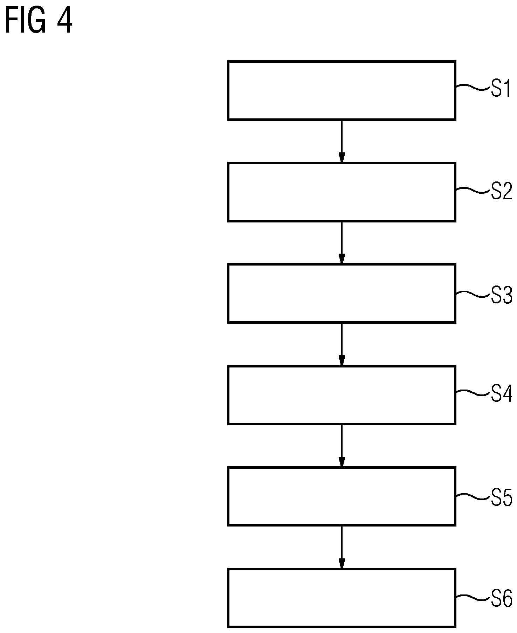

FIG 4 zeigt einen schematischen Verfahrensablauf eines Ausführungsbeispiels eines erfindungsgemäßen Verfahrens in einem Ablaufdiagramm. In einem ersten Schritt S1 erfolgt ein Berühren des virtuellen Bedienelements auf dem Berührbildschirm beispielsweise mit einem Finger. Der Berührbildschirm registriert eine derartige Berührung.

In einem anschließenden Schritt S2 erfolgt ein Verschieben des virtuellen Bedienelements an eine ausgewählte Position. Dabei zieht der Bediener das virtuelle Bedienelement an die gewünschte Position.

In einem Schritt S3 erfolgt das Bewegen der Objekttransporteinheit entsprechend der ausgelenkten Position. Dies bedeutet, dass automatisch erfasst wird, in welche Richtung ausgehend von der Ausgangsposition das virtuelle Bedienelement ausgelenkt wurde. Gegebenenfalls wird zusätzlich auch die Entfernung zwischen der ausgelenkten Position und der Ausgangsposition ermittelt, um abhängig davon die Geschwindigkeit der Objekttransporteinheit zu steuern. Mit der Richtung der Auslenkung auf dem Berührbildschirm lässt sich auch die Richtung des Transports der Objekttransporteinheit steuern.

Am Ende der gewünschten Bewegung lässt der Bediener das virtuelle Bedienelement gemäß Schritt S4 auf dem Berührbildschirm los. Dies bedeutet, dass der Bediener das virtuelle Bedienelement nicht weiter berührt und seinen Finger vom Bildschirm entfernt.

Das Loslassen des virtuellen Bedienelements entsprechend Schritt S4 wird von dem Berührbildschirm registriert und das virtuelle Bedienelement geht daraufhin optional in die Ausgangsposition gemäß Schritt S5 zurück.

Gleichzeitig mit dem Loslassen gemäß Schritt S4 oder aber bedingt durch das Zurückbewegen des Bedienelements in die Ausgangsposition gemäß Schritt S5 erfolgt entsprechend Schritt S6 eine Beendigung der Bewegung der Objekttransporteinheit. Diese Beendigung kann abrupt mit dem Loslassen der virtuellen Bedieneinheit erfolgen oder aber auch durch ein kontinuierliches Reduzieren der Geschwindigkeit.

## Patentansprüche

1. Untersuchungssystem (1) mit
- einer Untersuchungseinheit (2 bis 6), die einen Untersuchungsbereich aufweist,
- einer Objekttransporteinheit (7), mit der ein zu untersuchendes Objekt (8) in den und/oder aus dem Untersuchungsbereich transportierbar ist, und
- einem Berührbildschirm (12), der zur Steuerung einer Funktion und/oder zur Visualisierung eines Zustands der Untersuchungseinheit (2 bis 6) ausgebildet ist,
**dadurch gekennzeichnet, dass**
- der Berührbildschirm (12) ein virtuelles Bedienelement (13) aufweist, das manuell in eine Bewegung, eine Auslenkung oder eine Zielposition versetzbar ist, und
- die Objekttransporteinheit (7) in Abhängigkeit von der Bewegung, Auslenkung oder Zielposition des virtuellen Bedienelements (13) zu dem oder weg von dem Untersuchungsbereich bewegbar ist.

2. Untersuchungssystem nach Anspruch 1, wobei das virtuelle Bedienelement (13) als ausschließlich linear aus einer Ausgangsposition (16) heraus auslenkbares Graphikelement ausgebildet ist.

3. Untersuchungssystem nach Anspruch 1, wobei das virtuelle Bedienelement (13) als ausschließlich rotatorisch aus einer Ausgangsposition (16) heraus auslenkbares Graphikelement ausgebildet ist.

4. Untersuchungssystem nach einem der vorhergehenden Ansprüche, wobei eine Geschwindigkeit der Objekttransporteinheit (7) von einem Maß der Auslenkung des virtuellen Bedienelements (13) abhängt.

5. Untersuchungssystem nach einem der Ansprüche 2 bis 4, wobei das virtuelle Bedienelement (13) aus einer Auslenkung automatisch in die Ausgangsposition (16) zurückgeht, wenn das virtuelle Bedienelement (13) nicht mehr manuell berührt wird.

6. Untersuchungssystem nach einem der vorhergehenden Ansprüche, wobei der Berührbildschirm (12) direkt an der Untersuchungseinheit (2 bis 6) angebracht ist.

7. Bildgebendes Untersuchungssystem, das ein Untersuchungssystem (1) nach einem der vorhergehenden Ansprüche aufweist, wobei die Untersuchungseinheit (2 bis 6) dazu ausgebildet ist, von dem zu untersuchenden Objekt (8) in dem Untersuchungsbereich ein Bild zu gewinnen.

8. Bildgebendes Untersuchungssystem, das als MRT-System ausgebildet ist.

9. Verfahren zum Betreiben eines Untersuchungssystems (1) nach einem der vorhergehenden Ansprüche, wobei das virtuelle Bedienelement (13) des Berührbildschirms (12) manuell in eine Bewegung, Zielposition oder eine Auslenkung versetzt wird, und die Objekttransporteinheit (7) in Abhängigkeit von der Bewegung, Zielposition oder Auslenkung des virtuellen Bedienelements (13) zu dem oder weg von dem Untersuchungsbereich bewegt wird.
